Europäisches Patentamt

European Patent Office

Office européen des brevets

(19)

(11) Publication number: **0 252 186**
**A1**

(12) ## EUROPEAN PATENT APPLICATION

(21) Application number: 86201489.1

(51) Int. Cl.⁴: **A61F 13/16** , **A41B 13/02**

(22) Date of filing: 01.09.86

(30) Priority: 07.07.86 BE 216884

(43) Date of publication of application:
13.01.88 Bulletin 88/02

(84) Designated Contracting States:
**BE DE FR GB IT NL**

(71) Applicant: **Van Malderen, Paul**
**Kamerstraat 133**
**B-9360 Buggenhout(BE)**

(72) Inventor: **Van Malderen, Paul**
**Kamerstraat 133**
**B-9360 Buggenhout(BE)**

(74) Representative: **Pieraerts, Jacques et al**
**Bureau Gevers S.A. rue de Livourne 7, Bte. 1**
**B-1050 Bruxelles(BE)**

(54) **Bandage for private hygiene.**

(57) There is described a bandage for private hygiene, stick-on pad, nappy, or similar, comprised of a layer moisture-absorbing material which in a bandage or stick-on pad, forms a core, and at least one outer layer which in the same goods, forms a hydrophobic casing, in which between an outer layer (3) and said moisture-absorbing material (7), along that side of said latter material which is removed in the use position, from the body, there is provided a layer (5) breathing, moisture-impervious material.

Fig.1.

EP 0 252 186 A1

## Bandage for private hygiene

This invention relates to a bandage for private hygiene, a stick-on pad, a nappy, or similar, comprised of a layer of moisture-absorbing material which in a bandage or stick-on pad, forms a core, and at least one outer layer which in the same goods, forms a casing.

In such goods, as considered in the use position thereof, there is arranged on that side of the moisture-absorbing material layer which is removed from the body, a layer moisture-pervious material, generally polyethylene. The drawback of such an air-impervious material lies in such a material causing local perspiring, which is very disadvantageous.

The invention has thus also for object to obviate said drawback and to provide a bandage, a stick-on pad or a nappy which is secure from moisture seeping through, and offers a high comfort.

To obtain this according to the invention, there is provided between said outer layer and said moisture-absorbing material, along that side of said latter material which is removed in use position, from the body, a layer breathing, moisture-impervious material.

In a first possible embodiment, said layer breathing, moisture-impervious material, is comprised of a so-called non-woven material.

According to another possible embodiment, said layer breathing, moisture-impervious material, is comprised of a fabric or tissue.

In a possible, desired embodiment of the invention, said breathing, moisture-impervious material is so processed as to have the required features.

Other details and advantages of the invention will stand out from the following description, given by way of non limitative example and with reference to the accompanying drawings, in which:

Figure 1 is a lengthwise section through a stick-on pad according to the invention (1st embodiment).

Figure 2 is a lengthwise section through a stick-on pad according to the invention (2nd embodiment).

The figures pertain to a so-called stick-on pad, but it should be stressed that other goods which are used for similar purposes, may be made on the same basis, that is more particularly sanitary towels and nappies.

The stick-on pads 1 and 2 are comprised in the usual way of a casing 3 the end strips or cross edges of which are joined together with the usual methods, such connection being also used for connecting the further layers which will be described hereinafter.

There is first provided on that side which is removed from the body (such designations should always be considered in the object use position), a silicone paper 4 which as it is removed, lets see the one side of casing 3. The wordings casing and outer layer are equivalent. The wording casing refers more particularly to a bandage, while the wording outer layer is more suitable for nappies.

On the one side of said casing, adhesive strips are arranged for the fastening to underwear. This is also known and is not part of the invention principle.

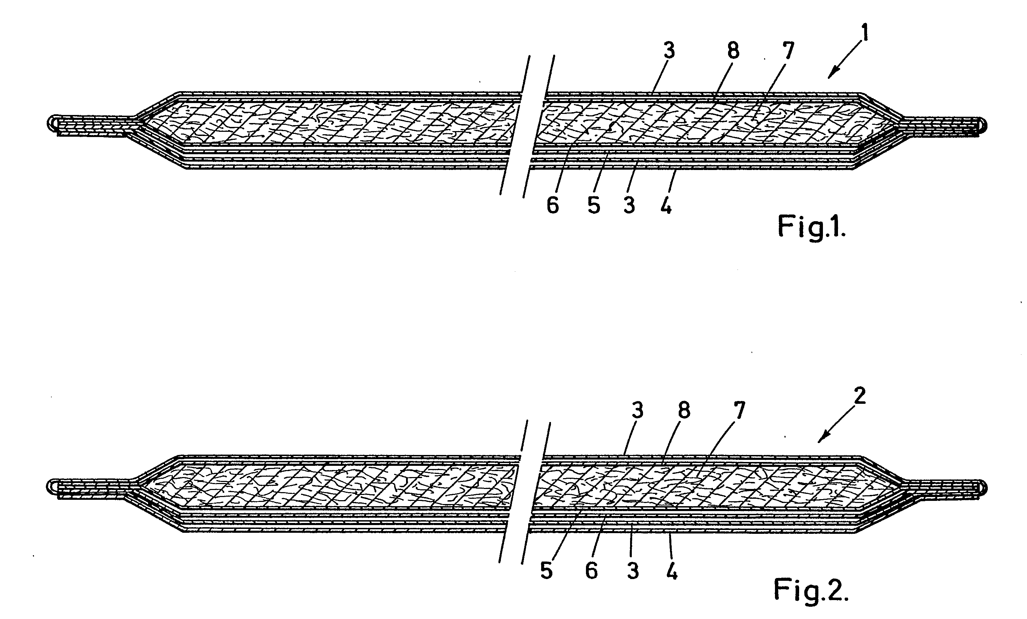

The following layer (fig. 1), this is essential for a clear understanding of the invention, is comprised of a layer 5 from a breathing, moisture-impervious material. Said layer 5 which is air-pervious but moisture-impervious, replaces the disturbing polyethylene which has been used up to now and serves as protecting layer. Due to said layer 5 being comprised of a material which "breathes", that is which lets air through but opposes the passage of moisture, there is thus obtained all the advantages from a good protecting layer which does however cause no local perspiring.

There is further a layer 6. Said layer is preferably comprised of cellulose paper, but it may also be made from other technically equivalent materials.

In a possible embodiment, said layer 6 may have been processed to serve as protecting layer. There is provided thereon the core 7 proper from moisture-absorbing material. The wording core refers more particularly to a stick-on pad or a bandage. In the case of a nappy, there is rather no mention of a core, but rather a layer moisture-absorbing material.

Still referring to figure 1, there is finally provided a layer which is comprised of a good moisture-absorbing material and is located between the absorbing core and the casing 3, along the use side.

In the embodiment as shown in figure 2, one encounters in sequence, starting from that side which faces the body: a casing 3 from non-woven material, a layer 6 from tissue, the breathing layer 5, the core 7 from moisture-absorbing material, then a layer 8 from tissue, and a non-woven material which is part of the casing 3 proper.

The layer 5 from breathing, moisture-impervious material may be a non-woven material. Such a non-woven material may be comprised of one of the following oil derivatives: polyester, viscose, polypropylene, nylon, or a technical equivalent thereof.

The material may have undergone a particular processing to obtain the required properties, whereby said layer can actually let through air but not moisture.

It is also possible to manufacture said layer 5 from a fabric. In such a case, the fabric is made from fibers which are in turn comprised of one of the following materials: cotton, nylon, polyester, wool, flax, viscose, or acryl.

There is meant by "breathing layer from moisture-impervious material", a product with a resistance to moisture perviousness which reaches at least 300 g/cm², while the air perviousness remains unhindered.

It appears clearly from the above description of a stick-on pad as shown by way of example in the figures, that the invention brings a substantial improvement as regards the comfort of such goods.

As already stated hereinabove, the same principle may be applied to bandages the structure of which has a substantial similarity with stick-on pads. Nappies or parts thereof may also be manufactured by using the same materials.

It must be understood that the invention is in no way limited to the above embodiments and that many changes may be brought thereto without departing from the scope of the invention as defined by the appended claims.

## Claims

1. Bandage for private hygiene, stick-on pad, nappy, or similar, comprised of a layer moisture-absorbing material which in a bandage or stick-on pad, forms a core, and at least one outer layer which in the same goods, forms a hydrophobic casing, in which between an outer layer (3) and said moisture-absorbing material (7), along that side of said latter material which is removed in the use position, from the body, there is provided a layer (5) breathing, moisture-impervious material.

2. Bandage for private hygiene, stick-on pad, nappy, or similar as defined in claim 1, in which said layer (5) breathing, moisture-impervious material, is comprised of a so-called non-woven material.

3. Bandage for private hygiene, stick-on pad, nappy, or similar as defined in claim 1, in which said layer (5) breathing, moisture-impervious material, is comprised of a fabric.

4. Bandage for private hygiene, stick-on pad, nappy, or similar as defined in any one of claims 1-3, in which said breathing, moisture-impervious layer (5) is comprised of a material which has been so processed as to have the required properties.

5. Bandage for private hygiene, stick-on pad, nappy, or similar as defined in claim 2, in which said non-woven material is an oil derivative selected among the following materials: polyester, viscose, polypropylene, nylon.

6. Bandage for private hygiene, stick-on pad, nappy or similar as defined in claim 3, in which said fabric is comprised of one of the following materials: cotton, nylon, polyester, wool, flax, viscose, acryl.

7. Bandage for private hygiene, stick-on pad, nappy, or similar as defined in any one of claims 1-6, in which between said core (7) from moisture-absorbing material and said layer (5) breathing, moisture-impervious material, there is provided a layer cellulose paper or technically equivalent material (6).

8. Bandage for private hygiene, stick-on pad, nappy, or similar as defined in any one of claims 1-6, in which between said core (7) from moisture-absorbing material and said layer cellulose material (6), there is provided a layer (5) breathing, moisture-impervious material, along that side away from the body.

9. Bandage for private hygiene as defined in any one of claims 1-8, in which said layer (5) breathing, moisture-impervious material has such properties that it has a resistance to moisture perviousness which reaches at least 300 g/cm², while the air perviousness remains unhindered.

Fig.1.

Fig.2.

European Patent Office

| | DOCUMENTS CONSIDERED TO BE RELEVANT | EP 86201489.1 | |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | EP - A1 - 0 104 906 (PERSONAL) <br> * Claims * <br> -- | 1,2,4,5 | A 61 F 13/16 <br> A 41 B 13/02 |
| A | EP - A2 - 0 098 083 (BOUSSAC) <br> * Claims; page 2, lines 26-38 * <br> -- | 1,2,4,5 | |
| A | US - A - 3 595 235 (JESPERSEN) <br> * Claims * <br> -- | 1,2 | |
| A | GB - A - 2 035 092 (COLGATE) <br> * Claims * <br> ---- | 1,2 | |
| | | | **TECHNICAL FIELDS SEARCHED (Int. Cl.4)** <br><br> A 61 F <br> A 41 B |

The present search report has been drawn up for all claims

| Place of search <br> VIENNA | Date of completion of the search <br> 12-10-1987 | Examiner <br> BECKER |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82